Europäisches Patentamt

European Patent Office

Office européen des brevets

(11) Veröffentlichungsnummer: **0 485 659 A1**

## EUROPÄISCHE PATENTANMELDUNG

(21) Anmeldenummer: **90121945.1**

(51) Int. Cl.⁵: **A61K 6/083**

(22) Anmeldetag: **16.11.90**

(43) Veröffentlichungstag der Anmeldung:
**20.05.92 Patentblatt 92/21**

(84) Benannte Vertragsstaaten:
**AT BE CH DE DK ES FR GB IT LI NL SE**

(71) Anmelder: **REISS, Siegfried**
**Jacobistrasse 35**
**W-6380 Bad Homburg(DE)**

(72) Erfinder: **REISS, Siegfried**
**Jacobistrasse 35**
**W-6380 Bad Homburg(DE)**

(74) Vertreter: **Weber, Dieter, Dr. et al**
**Dr. Dieter Weber und Dipl.-Phys. Klaus**
**Seiffert Patentanwälte**
**Gustav-Freytag-Strasse 25 Postfach 6145**
**W-6200 Wiesbaden 1(DE)**

(54) **Verfahren und Formmasse zur Herstellung von Formlingen für den Dentalbereich.**

(57) Ein Verfahren zur Herstellung von Formlingen für den Dentalbereich mit besseren mechanischen Werten und besserer Verträglichkeit, insbesondere von Dentalprothesen, deren Teilen oder kieferorthopädischen Apparaten, durch chemische Polymerisation von Harzsystemen auf Poly(meth)acrylatbasis mit Hilfe von Härtungskatalysatoren, ist dadurch gekennzeichnet, daß man Harzsysteme verwendet, die außer Härtungskatalysator zusätzlich wenigstens einen Photoinitiator enthalten, und nach der chemischen Polymerisation den Restmonomergehalt des Formlings durch anschließende Bestrahlung mit die Photoinitiatoren aktivierenden Strahlen unter Photopolymerisation vermindert.

EP 0 485 659 A1

Rank Xerox (UK) Business Services
(-/2.19/2.0)

EP 0 485 659 A1

Die Erfindung betrifft ein Verfahren zur Herstellung von Formlingen für den Dentalbereich, insbesondere von Dentalprothesen, deren Teilen oder kieferorthopädischen Apparaten, durch chemische Polymerisation von Harzsystemen auf Poly(meth)acrylatbasis mit Hilfe von Härtungskatalysatoren.

In der Zahntechnik werden für die Herstellung von Dentalprothesen, für Reparaturen und Unterfütterungen bekanntermaßen derartige Harzsysteme verwendet. Insbesondere handelt es sich dabei um Zweikomponentensysteme, von denen die eine Komponente ein Pulver und die andere eine Flüssigkeit, jeweils auf Acrylatbasis ist. Die Aushärtung der Formmassen erfolgt in unterschiedlicher Weise. Die Heißhärtung in der Zahntechnik bekannter Harzsysteme führt infolge der unterschiedlichen Ausdehnungskoeffizienten des Kunststoffes einerseits und der Modellmaterialien andererseits zu einer schlechten Paßgenauigkeit, so daß diese Härtungsmethode nur beschränkt anwendbar ist. Die ebenfalls bekannte Lichthärtung ist im allgemeinen nicht anwendbar, insbesondere nicht bei kompliziert geformten Formlingen, da bei diesen immer Schattenbereiche auftreten, in denen keine oder nur schlechte Aushärtung erfolgt. Nach dieser Methode gehärtete Formlinge besitzen schlechte mechanische Werte und sind nur begrenzt einfärbbar.

In der Zahntechnik haben sich daher vor allem autopolymerisierende Harzsysteme durchgesetzt, da diese eine bessere Paßgenauigkeit infolge der Anwendung niedrigerer Polymerisationstemperaturen ergeben und breitere Anwendungsmöglichkeiten zeigen. Diese autopolymerisierenden Systeme polymerisieren aufgrund chemischer Polymerisationsreaktionen, die mit Hilfe von Härtungskatalysatoren ausgelöst werden. Der Nachteil dieser autopolymerisierenden Harzsysteme besteht jedoch darin, daß die chemische Polymerisation während der Herstellung der Formlinge nicht vollständig abläuft, so daß nach Fertigstellung der Formlinge diese noch relativ hohe Restmonomergehalte besitzen, die erst nach langer Lagerzeit auf ein für den Patienten nicht mehr wahrnehmbares Maß herabgesetzt werden. Diese Restmonomergehalte verschlechtern die mechanischen Werte zu Beginn des Tragens einer Prothese, was in diesem Zeitraum zu einer Beschädigungsanfälligkeit führt. Diese ist besonders nachteilig, da der Patient gerade im Anfangszeitraum zu unsachgemäßer Behandlung der Prothese neigt, wenn ihm das Tragen der Prothese noch ungewohnt ist. Weiterhin führen die relativ hohen Restmonomergehalte im Bereich von mehreren Prozenten zu Schleimhautreizungen, unkontrollierter Verformung, hoher Wasseraufnahme und Verfärbungen.

Die der Erfindung zugrundeliegende Aufgabe bestand nun darin, diese bei der chemischen Autopolymerisation auftretenden Nachteile zu beseitigen und Formlinge für den Dentalbereich zu bekommen, die bessere mechanische Werte haben und vom Patienten besser vertragen werden.

Das erfindungsgemäße Verfahren mit den eingangs genannten Merkmalen ist, dadurch gekennzeichnet, daß man Harzsysteme verwendet, die außer Härtungskatalystor zusätzlich wenigstens einen Photoinitiator enthalten, und daß man nach der chemischen Polymerisation den Restmonomergehalt des Formlings durch anschließende Bestrahlung mit die Photoinitiatoren aktivierenden Strahlen unter Photopolymerisation vermindert.

Mit diesem Verfahren kann man bereits bei relativ kurzen Bestrahlungszeiten die Restmonomergehalte wesentlich unter 1 % herabsetzen, was die Verträglichkeit einer Prothese oder dergleichen für den Patienten erheblich verbessert und das Auftreten von Schleimhautreizungen verhindert. Außerdem werden durch die Herabsetzung des Restmonomeregehaltes von üblicherweise mehreren Prozenten am Ende der Formlingherstellung bis auf weniger als 1 % die mechanischen Werte, wie der Elastizitätsmodul und die Bruchfestigkeit sowie die bleibende Verformung wesentlich verbessert.

Die Bestrahlung erfolgt mit Licht im sichtbaren Bereich oder mit UV-Strahlung (UVA, UVB oder UVC) im Wellenlängenbereich von 100 bis 800 nm, vorzugsweise im UVA-Bereich mit etwa 315 bis 380 nm oder im sichtbaren Bereich.

Die Wirksamkeit der Bestrahlungsbehandlung kann durch Erwärmen erhöht werden, wobei jedoch darauf zu achten ist, daß herbei der Erweichungspunkt des Polymers nicht erreicht wird. Zweckmäßig wird die Bestrahlung bei einer Temperatur oberhalb Umgebungstemperatur bis zu etwa 60 °C durchgeführt.

Gute Ergebnisse bezüglich der Reduzierung des Restmonomergehaltes erreicht man bereits bei relativ kurzen Bestrahlungszeiten. Die Bestrahlung wird vorzugsweise während einer Zeitdauer von 0,1 bis 24 h durchgeführt. Die Auswahl der Bestrahlungsquelle, wie UV-Lampen, des Bestrahlungsquellenabstandes, der Bestrahlungszeit und der Temperatur können vom Fachmann ohne zusätzliche erfinderische Leistung vorgenommen und aufeinander abgestimmt werden, um die Verfahrensparameter der Bestrahlungsbehandlung so zu optimieren, daß auf die günstigste Weise der geringstmögliche Restmonomergehalt erreicht wird.

Die Erfindung betrifft auch spezielle Formmassen zur Durchführung des beschriebenen Verfahrens, wobei die bevorzugten Merkmale der Formmassen auch als bevorzugte Merkmale des obigen Verfahrens anzusehen sind.

Die erfindungsgemäßen Formmassen zur Durchführung des Verfahrens nach der Erfindung auf der Basis eines Harzsystems auf Poly(meth)acrylatbasis sind dadurch gekennzeichnet, daß sie zusätzlich zu wenigstens einem chemischen Härtungskatalysator wenigstens einen Photoinitiator enthalten.

Wenn in der Beschreibung und den Ansprüchen von Harzsystmen auf Poly(meth)acrylatbasis die Rede ist, bedeutet dies, daß diese Harzsysteme im auspolymerisierten Zustand wenigstens ein Polyacrylat und/oder Polymethacrylat enthalten. Diese Harzsysteme können Einkomponentensysteme sein, sind aber vorzugsweise Zweikomponentensysteme und bestehen, wie bekannt, aus einer Pulverkomponente und einer flüssigen Komponente. Die Einkomponentensysteme sind im Regelfall warmhärtend, die Zweikomponentensysteme können kalthärtend oder warmhärtend sein.

Die Pulverkomponente besteht zweckmäßig aus wenigstens einem Homopolymer oder Copolymer von $(C_1-C_4$-Alkyl)-acrylat und/oder - $(C_1-C_4$-Alkyl)-methacrylat, wie insbesondere Methylmethacrylat, Ethylmethacrylat oder Butylmethacrylat. Im Falle der kalthärtenden Zweikomponentensysteme enthält diese Komponente zweckmäßig Katalysator, insbesondere Peroxidkatalysator, ein Amin-Peroxid-System oder ein Barbituratsystem. Die als Katalysatoren verwendeten organischen Peroxide sind die üblichen hierfür verwendeten Peroxide, wie z. B. Benzoylperoxid. Diese sind zweckmäßig in einer Menge von 0,1 bis 3 Gew.-%, bezogen auf das Gewicht der Pulverkomponente, enthalten.

Im Falle warmhärtender Zweikomponentensysteme dieser Kategorie enthält gewöhnlich nur Pulverkomponente Härtungskatalysator, ist aber im übrigen wie bei den kalthärtenden Systemen aufgebaut.

Die flüssige Komponente des Zweikomponentensystems enthält zweckmäßig als Hauptbestandteil wenigstens ein $C_1-C_{14}$-Alkylmethacrylat, gegebenenfalls mit anderen Comonomeren, und als Vernetzer ein Di- oder Tri-Alkylmethacrylatoder Di- oder Tri-[urethan-(meth)acrylat)]. In kalthärtenden Systemen enthält diese Komponente zweckmäßig einen üblichen Aminkatalysator und als Beschleuniger Kobalt- und/oder Kupferionenund/oder Chloridionen. In warmhärtenden Systemen enthält diese Komponente gewöhnlich weder einen Aminkatalysator, noch einen Beschleuniger.

Allgemein ist zu sagen, daß die erfindungsgemäß verwendeten Formmassen in üblicher Weise zusammengesetzt sein können, mit Ausnahme der Tatsache, daß zusätzlich zu chemischem Härtungskatalystor wenigstens ein Photoinitiator enthalten ist, um anschließend an die chemische Polymerisation eine Photopolymerisation zur Reduzierung des Restmonomergehaltes zu ermöglichen.

Außer den am üblichsten verwendeten Homopolymeren auf Acrylatbasis kommen natürlich auch Copolymere in Betracht, insbesondere solche mit mindestens 80 Gew.-% Alkylmethacrylateinheiten. Die Comonomereinheiten der Copolymere sind beispielsweise solche von Styrol oder Styrolderivaten, von Vinylacetat, von Acrylsäure oder Methacrylsäure, von gegebenenfalls substituiertem Acrylamid oder Metharcrylamid, von Acrylnitril oder Methacrylnitril, von Ethylenglycoldimethacrylat, von Triallylcyanurat, von Divinylbenzol oder von Allylacrylat.

Als Photoinitiatoren können unterschiedliche Systeme verwendet werden, die nach dem Stand der Technik an sich bekannt sind. Auch weiß man von diesen Photoinitiatoren aus der Literatur, bei welchen Wellenlängen der einfallenden Strahlung sie aktiviert werden. Beispiele erfindungsgemäß brauchbarer Photoinitiatoren sind Benzoinalkylether, wie Benzoinethylether oder Benzoinbutylether, Ketale von 4- - Diketonen, wie Benzilketale, insbesondere Benzildimethylketal, oder Diethoxyacetophenon, Benzophenonsysteme, wie Benzophenon selbst, gegebenenfalls zusammen mit einem tertiären Amin, Arylketone, wie Phenyl-2-hydroxy-2-propylketon, 2-Methyl-1-[4-(methylthio)-phenyl]-2-morpholinopropanon-1 oder 1-(4-Isopropylphenyl)-2-hydroxy-2-methylpropan-1-on oder polyzyklische aromatische Ketone, wie 2-Ethylanthrachinon, Xanthon, Thioxanthon oder 2-Chlorthioxanthon, jeweils gegebenenfalls in Verbindung mit einem tertiären Amin, halogenierte Ketone, wie 4-Tertiärbutyltrichloracetophenon, Acylphosphinoxide, wie 2,4,6-Trimethylbenzoyldiphenylphosphinoxid, Campherchinon und dessen Derivate, Dialkoxyacetophenone oder - Acyloximester.

Der Photoinitiator ist bei Zweikomponentensystemen zweckmäßig in der flüssigen Komponente enthalten. Gewöhnlich wird er in einer Menge von 0,01 bis 5 Gew.-%, vorzugsweise von 0,1 bis e Gew.-%, bezogen auf die in dem Harzsystem enthaltene Monomermenge, verwendet.

Im Dentalbereich ist es allgemein erwünscht, daß der Photoinitiator keine Vergilbung ergibt und wird daher unter anderem auch nach diesem Gesichtspunkt ausgewählt.

Beispiel und Vergleichsbeipsiel

Ein Zweikomponentensystem wurde aus 10 Gewichtsteilen einer Pulverkomponente und 5,5 Gewichtsteilen einer flüssigen Komponente zusammengesetzt. Die Pulverkomponente bestand aus einem handelsüblichen Polymethylmethacrylatcopolymer mit Benzoylperoxid und einem Barbitursäurederivat als Katalysatoren sowie Farbpigmenten. Die flüssige Komponente bestand aus Methylmethacrylat, Vernetzer und quaternärem Ammoniumsalz.

Das Gemisch wurde in einer Küvettenform zu Stäben einer Dicke von 3 mm, einer Breite von 10 mm und einer Länge von 70 mm geformt, indem das Gemisch 1 min nach dem Anmischen in die Form gegossen wurde. Die Polymerisation erfolgte nach ca. 5 min in einem handelsüblichen Druckpolymerisationsgerät unter einem Druck von 4 bar und einer Wassertemperatur von 40 °C während 30 min.

In einem Parallelversuch wurden der flüssigen Komponente 1,5 Gew.-%, bezogen auf das Gewicht dieser Komponente, 1-(4-Isopropylphenyl)-2-hydroxy-2-methylpropan-1-on als Photoinitiator zugesetzt. Im übrigen wurden aus dieser erfindungsgemäß hergestellten Formmasse Stäbchen wie oben hergestellt. Die letzteren Stäbchen mit einem Gehalt an Photoinitiator wurden 1 h nach der Polymerisation mit einer UVA-Lampe (Dulux S 9 W/78 von Phillips - Wellenlängenbereich 315 bis 380 nm) im Abstand von 40 mm während der in der nachfolgenden Tabelle aufgeführten Seiten betrahlt.

Für beide Versuche wurde der Restmonomergehalt nach 24 h Lagerung bzw. Bestrahlung bestimmt. Die außerhalb des Erfindungsgedankens hergestellten Stäbchen ohne Photoinitiator enthielten nach 24 h Lagerung einen Restmonomergehalt an Methylmethacrylat von 4,4 Gew.-%. Die erfindungsgemäß hergestellten Stäbchen mit Photoinitiator enthielten nach 24stündiger Bestrahlung einen Restmonomergehalt von nur 0,6 Gew.-% Methylmethacrylat.

Außerdem wurden für die Teststäbchen beider Versuche der Elastizitätsmodul und die Bruchfestigkeit bestimmt sowie eine bleibende Verformung visuell ermittelt. Die Ergebnisse dieser Versuche waren folgende:

## Tabelle

| Teststäbchen | Lagerzeit, h | E-Modul $N/mm^2$ | Bruchfestigkeit $N/mm^2$ |
|---|---|---|---|
| ohne | 4 | 1636 | 58 |
| Photoinitiator | 6 | 1802 | 62 |
| | 24 | 2398 | 80 |

| Teststäbchen | Bestrahlungs zeit, h | E-Modul $N/mm^2$ | Bruchfestighkeit $N/mm^2$ |
|---|---|---|---|
| mit | 4 | 2665 | 86 |
| Photoinitiator | 6 | 3062 | 87 |
| | 24 | 3155 | 86 |

Die ohne Photoinitiator hergestellten Stäbchen außerhalb des Erfindungsgedankens zeigten starke bleibende Verformung ohne Bruch der Prüfkörper bei einer Meßtemperatur von 27 °C. Die erfindungsgemäß mit Photoinitiator hergestellten Stäbchen zeigten nur sehr geringe bleibende Verformung unter den gleichen Bedingungen.

**Patentansprüche**

1.  Verfahren zur Herstellung von Formlingen für den Dentalbereich, insbesondere von Dentalprothesen, deren Teilen oder kieferorthopädischen Apparaten, durch chemische Polymerisation von Harzsystemen auf Poly(meth)acrylatbasis mit Hilfe von Härtungskatalysatoren, **dadurch gekennzeichne**t, daß man Harzsysteme verwendet, die außer Härtungskatalysator zusätzlich wenigstens einen Photoinitiator enthalten, und nach der chemischen Polymerisation den Restmonomergehalt des Formlings durch anschließende Bestrahlung mit die Photoiniatoren aktivierenden Strahlen unter Photopolymerisation vermindert.

2.  Verfahren nach Anspruch 1, **dadurch gekennzeichnet**, das man den Restmonomergehalt durch Bestrahlung bei Wellenlängen von 100 bis 800 nm, vorzugsweise im UVA-Bereich und im sicht baren Lichtbereich, vermindert.

3.  Verfahren nach Anspruch 1 oder 2, **dadurch gekennzeichnet**, daß man während der Bestrahlung auf eine Temperatur bis zu 60 ° C erwärmt.

4.  Verfahren nach einem der Ansprüche 1 bis 3, **dadurch gekennzeichnet**, daß man die Bestrahlung während 0,1 bis 24 h durchführt.

5.  Formmasse zur Durchführung des Verfahrens nach einem der Ansprüche 1 bis 4 aus einem Harzsystem auf Poly(meth)acrylatbasis, **dadurch gekennzeichnet**, daß sie zusätzlich zu wenigstens einem chemischen Härtungskatalysator wenigstens einen Photoinitiator enthält.

6.  Formmasse nach Anspruch 1, **dadurch gekennzeichnet**, daß das Harzsystem aus einem Zweikomponentensystem besteht, dessen eine Komponente ein Pulver eines Homo- oder Copolymers $(C_1-C_4-$Alkyl)-(meth)acry-lat, gegebenenfalls mit Katalysatorgehalt, und dessen andere Komponente flüssiges $C_1-C_{14}$-Alkylmethacrylat, gegebenenfalls mit anderen Comonomeren mit einem Vernetzer und gegebenenfalls mit einem Katalysator und/oder Beschleuniger ist.

7.  Formmasse nach Anspruch 5 oder 6, **dadurch gekennzeichnet**, daß sie als Härtungskatalysator ein organisches Peroxid, Amin und/oder Barbitursäurederivat enthält.

8.  Formmasse nach einem der Ansprüche 5 bis 7, **dadurch gekennzeichnet**, daß sie als Photoinitiator einen Benzoinalkylester, ein Ketal eines -Diketons, ein Benzophenon, ein Arylketon, ein halogeniertes Keton, ein Acylphosphinoxid, ein Campherchinonderivat, ein Dialkoxyacetophenon oder einen - Acyloximester enthält.

9.  Formmasse nach einem der Ansprüche 6 bis 8, **dadurch gekennzeichnet.**, daß sie den Photoinitiator in der flüssigen Komponente enthält.

10. Formmasse nach einem der Ansprüche 5 bis 9, **dadurch gekennzeichnet**, daß sie den Photoinitiator in einer Menge von 0,01 bis 5 Gew.-%, vorzugsweise von 0,1 bis 3 Gew.-%, bezogen auf die in ihr enthaltene Monomermenge, enthält.

11. Formmasse nach einem der Ansprüche 6 bis 10, **dadurch gekennzeichnet**, daß die Pulverkomponente Polymethylmethacrylat enthält.

12. Formmasse nach einem der Ansprüche 6 bis 11, **dadurch gekennzeichnet**, daß die flüssige Komponente Methylmethacrylat enthält.

13. Formmasse nach einem der Ansprüche 6 bis 12, **dadurch gekennzeichnet**, daß die flüssige Komponente als Vernetzer wenigstens ein Di- oder Tri(alkylmethacrylat), vorzugsweise mit 1 bis 4 Kohlenstoffatomen in der Alkylgruppe, oder Di- oder Tri-(urethanmethacrylat) oder Di- oder Tri-(urethanacrylat) enthält.

14. Formmasse nach einem der Ansprüche 6 bis 13, **dadurch gekennzeichnet**, daß sie als Beschleuniger Kobalt- und/oder Kupfer-Ionen und/oder Chloridionen enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| E | DE-A-3 928 987 (S. REISS) <br> * Insgesamt * <br> --- | 1-14 | A 61 K 6/083 |
| X | EP-A-0 195 224 (IVOCLAR) <br> * Patentansprüche; Beispiele 1-5 * <br> --- | 1-13 | |
| X | DE-A-2 914 537 (SANKIN) <br> * Patentansprüche * <br> --- | 1-13 | |
| X | DE-A-3 709 881 (G.C. DENTAL IND. CORP.) <br> * Seite 4, Zeile 59 - Seite 5, Zeile 30; Beispiele * <br> --- | 1-13 | |
| A | DE-A-3 610 683 (W. HEYNOLD) <br> * Patentanspruch 3 * <br> --- | 7,8 | |
| A | GB-A-2 075 035 (COLGATE- PALMOLIVE) <br> * Seite 2, Zeile 59 - Seite 3, Zeile 5 * <br> ----- | 6-14 | |

**RECHERCHIERTE SACHGEBIETE (Int. Cl.5)**

A 61 K

Der vorliegende Recherchenbericht wurde für alle Patentansprüche erstellt

| Recherchenort | Abschlußdatum der Recherche | Prüfer |
|---|---|---|
| DEN HAAG | 04-07-1991 | COUSINS-VAN STEEN G.I.L. |